# EUROPÄISCHE PATENTSCHRIFT

(11) **EP 3 649 092 B1**
(45) Veröffentlichungstag und Bekanntmachung des Hinweises auf die Patenterteilung: **25.02.2026**
(21) Anmeldenummer: 18739788.0
(22) Anmeldetag: 02.07.2018
(51) Int. Cl.: C03C 23/00, A61M 5/31

(54) **GLASZYLINDER FÜR EINE KOLBEN-ZYLINDER-ANORDNUNG MIT VERMINDERTER REIBUNG UND VERFAHREN ZUR BEHANDLUNG EINES GLASZYLINDERS FÜR EINE KOLBEN-ZYLINDER-ANORDNUNG**
GLASS CYLINDER FOR A PISTON-CYLINDER ASSEMBLY WITH REDUCED FRICTION, AND METHOD FOR TREATING A GLASS CYLINDER FOR A PISTON-CYLINDER ASSEMBLY
CYLINDRE DE VERRE POUR UN ENSEMBLE PISTON-CYLINDRE À FRICTION RÉDUITE ET PROCÉDÉ DE TRAITEMENT D'UN CYLINDRE DE VERRE POUR UN ENSEMBLE PISTON-CYLINDRE

(30) Priorität: 05.07.2017 DE 102017114959
(43) Veröffentlichungstag der Anmeldung: 13.05.2020
(73) Patentinhaber: SCHOTT Pharma AG & Co. KGaA, 55122 Mainz (DE); SCHOTT Pharma Schweiz AG, 9000 St. Gallen (CH)
(72) Erfinder: RUDIGIER-VOIGT, Eveline, 55128 Mainz (DE); DJORDJEVIC-REISS, Jovanna, 55130 Mainz (DE); SCHNEIDER, Thorsten, 55299 Nackenheim (DE); GEIGER, Joerg, VERSTORBEN (DE); GRIGAS, Daniel, 63654 Büdingen (DE)
(74) Vertreter: Blumbach · Zinngrebe Patentanwälte PartG mbB
(86) Internationale Anmeldenummer: PCT/EP2018/067754
(87) Internationale Veröffentlichungsnummer: WO 2019/007876

(56) Entgegenhaltungen:
- WO-A1-2018/157097
- US-A1- 2003 023 206
- US-A1- 2008 044 588
- US-A1- 2012 251 748
- US-A1- 2013 171 334
- US-A1- 2016 052 821

## Beschreibung

Die Erfindung betrifft allgemein Anordnungen mit Zylinder und darin laufendem Kolben. Im Speziellen betrifft die Erfindung solche Anordnungen mit einem Glaszylinder.

Im Maschinenbau sind Kolben-Zylinder-Anordnungen weit verbreitet. Typischerweise wird hier Metall als Material sowohl für den Kolben, als auch den Zylinder verwendet. Um den Kolben dichtend im Zylinder gleiten lassen zu können, werden Schmiermittel verwendet.

In speziellen Bereichen der Technik kommen auch Kunststoffe zum Einsatz. Einige Kunststoffe haben den Vorzug, dass sie nur geringe Reibung verursachen, so dass auch schmiermittelfreie Anordnungen möglich sind. Ein Beispiel hierfür sind Kunststoffspritzen zur Verabreichung von Medikamenten.

Neben Kunststoffspritzen werden auch Glasspritzen eingesetzt, diese oft auch in Form vorbefüllter Spritzen. Vorfüllbare Spritzen bieten gegenüber der herkömmlichen Kombination aus Fläschchen (Vials) und Einwegspritze mehrere Vorteile in der pharmazeutischen Verpackung, so z. B. die genaue Medikamentendosierung, die Gewährleistung der Sterilität, die Zeitersparnis in Notsituationen, Benutzerfreundlichkeit und nicht zuletzt weniger Abfall und geringere Umweltbelastung. Eine vorgefüllte Spritze ist üblicherweise als ein dreiteiliges Gerät bestehend aus den Komponenten (a) Spritzenzylinder mit angepasster Nadel oder einem sog. Luer-Adapter, (b) Gummistopfen der die Behälter-Verschlussintegrität liefert bzw. gewährleistet und (c) einer Kunststoff-Kolbenstange, die verwendet wird, um den Stopfen durch den Glaszylinder vorzuschieben und damit das Medikament zu verabreichen, definiert. Vorfüllbare Glasspritzen, werden vom Primärverpackungshersteller gewaschen, silikonisiert, sterilisiert und verpackt. Somit enthalten alle Spritzen auch eine nicht sichtbare vierte Komponente, das Schmiermittel, das oft übersehen wird und einen großen Einfluss auf die Spritzenfunktion und die Arzneimittelstabilität haben kann.

Glasspritzen bieten den Vorteil einer hohen Hitzebeständigkeit, so dass diese einfach sterilisiert werden können. Zudem ist die Permeabilität für Gase im Gegensatz zu Kunststoffen sehr gering. In der Spritze lassen sich daher Medikamente auch über längere Zeiträume lagern. Glas ist andererseits ein sprödharter Werkstoff, bei dem schnell ein Klemmen oder Festfressen mit Abriss von Glaspartikeln erfolgen kann, wenn nicht ausreichend geschmiert wird. Es ist daher bekannt, Glasspritzen innenseitig mit einer reibvermindernden Beschichtung oder einem Schmiermittel zu versehen. Die Gleitfähigkeit wird heutzutage branchenüblich über einen kontrollierten Silikonisierprozess gewährleistet, über welchen das Silikonprofil individuell an die jeweiligen Kundenanforderungen angepasst werden kann. Üblicherweise werden dazu Silikonöle eingesetzt.

Mehrere Aspekte dieser Spritzen-Silikonisierung stellen eine Herausforderung für ihre Verwendung als vorgefüllte Spritzen dar: So können in speziellen Fällen bereits wenige Mengen von ungebundenem Silikonöl bzw. Silikonpartikeln das Medikament signifikant beeinflussen und damit die Wirksamkeit herabsetzen, was besonders in Bezug auf Biotech-Pharmazeutika zu vermeiden ist, da es in diesem Fall zu einer Aggregation spezieller Proteine kommen kann. Aus den genannten Gründen (Vermeidung von Silikonpartikeln in Medikamenten und völliger Verzicht von Silikon für hoch-sensitive Medikamente) ist es wünschenswert, mindestens den Silikongehalt signifikant zu verringern bzw. auf Silikon gänzlich zu verzichten bei gleichbleibenden Reibeigenschaften und Container Closure Integrity.

Die Silikonisierung des pharmazeutischen Primärpackmittels, genauer des Spritzenzylinders ist heutzutage ein äußerst wichtiger Aspekt der Herstellung von sterilen, vorfüllbaren Glasspritzen, da die funktionelle Wechselwirkung des Glaskörpers und des Stopfens entscheidend für die Effizienz des Gesamtsystems ist. Sowohl unzureichende als auch übermäßige Silikonisierung können in diesem Zusammenhang wie bereits beschrieben Probleme verursachen. Deshalb ist ein Trend - durch Einsatz moderner Applikationstechnologien - eine extrem gleichmäßige Verteilung von Silikonöl in Glasspritzen einhergehend mit drastisch reduzierten Mengen an Silikonöl zu erreichen. Eine weitere Möglichkeit zur Minimierung der Menge an freiem Silikonöl in einer Spritze ist die thermische Fixierung des Silikonöls auf der Glasoberfläche in einem Prozess, der als Einbrennsilikonisierung - so genanntes 'baked on silicone' - bezeichnet wird. Silikonölfreie bzw. schmiermittelfreie Glaskörper (z. B. Spritzen oder Karpulen) sind im Hinblick auf die genannten Nachteile und den stetig wachsenden Markt an hoch sensiblen Biotech-Pharmazeutika von großem Interesse. Biotech-Pharmazeutika sind eine wichtige Klasse von Arzneimitteln (z. B. Insulin, Impfstoffe, Antikörper, Blutprodukte, Hormone, Zytokine), welche mit großem technologischen Aufwand sowie aufwändigen Entwicklungs- und Fertigungsmethoden hergestellt werden und sehr empfindlich auf Gleitmittel (z. B. Silikonöl) reagieren, weshalb die Herausforderungen an die konventionelle Spritzentechnologie steigen.

Für Kunststoffspritzen (COC oder COP) wird bereits die Gleitfähigkeit spezieller Fluorpolymerbeschichtungen auf speziell entwickelten Kolbenstopfen genutzt, um die Silikonisierung von diesen Spritzen komplett überflüssig zu machen. Dieser Ansatz ist für Glasoberflächen jedoch nur teilweise übertragbar, da sich die Materialeigenschaften von Kunststoffen und Glas signifikant unterscheiden. Glasoberflächen unterliegen gegenüber Kunststoffoberflächen einer höheren Dynamik bzw. Wechselwirkung mit der sie umgebenden Atmosphäre in Form von Oberflächenreaktionen. Unter diesen Gesichtspunkten müssen spezielle Überlegungen für vorgefüllte Glasspritzenvorrichtungen und ähnliche Vorrichtungen (z. B. Karpulen) unternommen werden, um deren Komponenten steril zu halten und deren Stabilität und die optische Klarheit mit Transport und Lagerung bis zu einigen Jahren zu gewährleisten. Speziell die Reibung zwischen Stopfenmaterial und Spritzenkörper kann erheblich sein, weshalb die Notwendigkeit besteht, die Reibung zwischen Stopfen und Glaszylinder ohne die Verwendung von Ölen oder anderen Schmiermitteln stabil über Zeitraum der Lagerung bis hin zur Verwendung zu reduzieren.

Die US 4767414 A beschreibt ein Verfahren zum Reduzieren der statischen und dynamischen Reibung zwischen Gleitflächen durch Aufbringen eines Gleitfilms auf zumindest eine der Oberflächen. Dabei wird ein niedermolekulares Silikonöl auf eine der Oberflächen aufgetragen. Das Silikonöl und die Oberfläche werden plasmabehandelt.

Die US 2004/0231926 A1 beschreibt ein Verfahren zur Herstellung einer Gleitschicht, bei dem die Gleitschicht bei Atmosphärendruck unter anderem unter Verwendung eines Atmosphärendruck-Plasmas ausgehärtet wird. Neben Silikonöl-basierten Schichten können auch Perfluorpolyether-basierte Gleitschichten hergestellt werden. Die Losbrechkraft, beziehungsweise die Haftreibung der letzteren Schichten erweist sich allerdings als höher verglichen mit ausgehärteten Silikonöl-Schichten. Zudem kann es bei einer Atmosphärendruck-Behandlung, insbesondere bei Atmosphärendruck-Plasma-Behandlung, zum erhöhten Eintrag von Gasen, insbesondere auch von Reaktionsprodukten des Plasmas in die Schicht kommen.

Aus der WO 2011/029628 A1 ist es weiterhin bekannt, eine silikonfreie Gleitschicht für eine Glasspritze mit besonders niedriger Reibung durch Vernetzung eines silikonfreien organischen Fluids in einer Niederdruck-Glimmentladung vorzunehmen.

Auch bei Kunststoffspritzen wird auf Silikon-Gleitschichten zurückgegriffen. Solche Gleitschichten sind beispielsweise aus der US 2012/277686 A1 oder US 2008/071228 A1 bekannt. Ein weiteres Verfahren zur Verminderung der Reibung des Kolbens in Glasspritzen wird in der US 2008/044588 beschrieben.

Zumindest für bestimmte Wirkstoffe wäre es wünschenswert, wenn eine Glasspritze ohne Gleitschicht auf dem Glaszylinder verwendet werden könnte, der dennoch gute Gleiteigenschaften aufweist. Auch für andere Kolben-Zylinder-Anordnungen mit Glaszylindern wäre dies von Vorteil.

Der Erfindung liegt demgemäß die Aufgabe zugrunde, eine entsprechende Kolben-Zylinder-Anordnung und einen Glaszylinder für eine solche Anordnung bereitzustellen.

Diese Aufgabe wird durch den Gegenstand der unabhängigen Ansprüche gelöst. Vorteilhafte Ausgestaltungen der Erfindung sind in den jeweiligen abhängigen Ansprüchen angegeben.

Zur Lösung dieser Aufgabe sieht die Erfindung ein Verfahren zur Behandlung eines Glaszylinders für eine Kolben-Zylinder-Anordnung zur Verminderung der Reibung des Kolbens an der Zylinder-Innenwandung vor, bei welchem bei welchem im von der Zylinder-Innenwandung begrenzten Innenraum die Oberflächenenergie des Glases erhöht und dadurch der Kontaktwinkel des Glases mit Wasser abgesenkt wird durch
- eine Gasentladung, welche auf das Glas an der Zylinder-Innenwandung einwirkt und durch ein elektrisches oder elektromagnetisches Feld erzeugt wird, oder
- durch Einwirkung von Ozon auf die Glasoberfläche, wobei

nach der Einwirkung der Gasentladung, des Plasmas oder des Ozons das Glas an der Zylinder-Innenseite mit Wasser beaufschlagt wird,
wobei die Oberfläche der Zylinder-Innenseite durch das Glas des Glaszylinders gebildet wird, und wobei der Kontaktwinkel dieser Oberfläche mit Wasser kleiner als 15° beträgt und die Variation des Kontaktwinkels des Glases mit Wasser in Längsrichtung von Ende zu Ende des Glaszylinders weniger als 5° beträgt.

Besonders bevorzugt wird die Erfindung für Glasspritzen eingesetzt.

Zur Erzielung einer Gleitwirkung, welche eine einfache Bewegung des Kolbens im Glaszylinder ermöglicht, wird der Reibkoeffizient bisher in Richtung großer Wasserkontaktwinkel verändert. Dabei wird die Oberfläche des Glaszylinders hydrophob. Der ebenfalls hydrophobe Gleitpartner, in diesem Falle der Stopfen, beziehungsweise Kolben kann mit wenig Kraft gut über die Oberfläche gleiten. Dieser Schritt wird im Stand der Technik beispielsweise durch Silikonisierung der Glasoberfläche erreicht.

Mit der Erfindung wird der entgegengesetzte Weg eingeschlagen, indem eine hydrophile Glasoberfläche geschaffen wird. Durch die Benetzungseigenschaften der Oberfläche bildet sich ein Wasserfilm aus und der Stopfen gleitet mit wenig Kraft auf diesem Wasserfilm.

Bei dem Wasser handelt es sich gemäß einer Ausführungsform nicht um Wasser eines in von der Kolben-Zylinder-Anordnung aufgenommenen Wirkstoff-Zubereitung. Vielmehr ist die Beladung mit Wasser nur vorübergehend. Typischerweise wird der Glaszylinder nach der Beladung mit Wasser in leerem Zustand zwischengelagert, bis dieser mit der zu verabreichenden Wirkstoff-Zubereitung, die oft ebenfalls auf Wasserbasis ist oder zumindest Wasser enthält, befüllt wird. Gemäß einer anderen Ausführungsform kann das Beaufschlagen mit Wasser aber auch durch Befüllen mit einer wasserhaltigen Wirkstoff-Zubereitung erfolgen.

Wenn die Erhöhung der Oberflächenenergie des Glases durch Einwirkung von Ozon auf die Glasoberfläche hervorgerufen wird, wird das Ozon vorzugsweise durch zumindest einen der Prozesse:
- eine Gasentladung in einem Sauerstoff enthaltenden Gas,
- eine Bestrahlung mit ionisierenden Strahlen
- eine UV-Bestrahlung
erzeugt.

Die Gasentladung erfolgt in bevorzugter Ausgestaltung der Erfindung in einem Sauerstoff enthaltenden Gas, insbesondere reinem Sauerstoff oder einem Gasgemisch, wie etwa ein Sauerstoff-Stickstoff-Gemisch oder ein Sauerstoff-Edelgas-Gemisch. Es hat sich gezeigt, dass auch Luft als Prozessgas geeignet ist, um die erfindungsgemäß vorgesehene Absenkung des Kontaktwinkels mit Wasser hervorzurufen.

In einer besonderen Ausführungsform wird keine Niederdruck-Gasentladung durchgeführt. Vielmehr kann die Gasentladung bei einem Gasdruck von mindestens 100 Millibar erfolgen. Besonders bevorzugt wird eine Gasentladung unter Atmosphärendruck. Auf diese Weise kann eine Abdichtung des Glaszylinders und eine Evakuierung entfallen. Gemäß noch einer Ausführung erfolgt die Gasentladung bei niedrigem Druck von höchstens 10 mBar. Ein geeigneter Druck liegt beispielsweise bei 0,3 Millibar.

Besonders geeignet ist eine Gasentladung in Form einer Korona-Entladung. Diese Entladung ermöglicht es, die Behandlung auch unter Atmosphärendruck durchzuführen und dabei eine homogene Einstellung der Oberflächenenergie an der Innenseite des Glaszylinders einzustellen. Bei einer Variante der Korona-Behandlung kann auch Ozon erzeugt werden. Ozon lässt sich weiterhin auch in einer Gasentladung, wie etwa einer Plasmaentladung oder durch Einstrahlung von UV-Licht erzeugen.

Gemäß noch einer bevorzugten Ausführungsform umfasst die Gasentladung eine Plasmabehandlung, beziehungsweise das Erzeugen eines Plasmas.

Die Plasmabehandlung wird im Sinne dieser Erfindung als besondere Form der Gasentladung bezeichnet, bei der die Entladung zur Bildung eines Plasmas führt. Für die Plasmabehandlung können elektromagnetische Wechselfelder zur Erzeugung der Gasentladung in Form eines Plasmas eingesetzt werde. Geeignete Wechselfelder sind RF- HF- oder Mikrowellenfelder. Der Begriff einer Gasentladung ist allgemein im Sinne dieser Offenbarung nicht auf Entladungen in Gleichspannungsfeldern begrenzt.

Für das Beladen mit Wasser ergeben sich verschiedene Möglichkeiten. Beispielsweise könnte der Glaszylinder mit Wasser durchgespült oder in ein Wasserbad eingetaucht werden. Sprühen oder Ultraschall-Vernebeln sind weitere Möglichkeiten. Gemäß einer bevorzugten Ausführungsform des Verfahrens umfasst das Beladen mit Wasser das Einführen von Wasserdampf in den Innenraum des Glaszylinders. Dies bietet den Vorteil, dass ein Trocknungsschritt zur Entfernung von verbleibendem Wasser nach Entleerung entfallen kann. Zudem kann mit der Zuführung von Wasserdampf vermieden werden, dass eine Kontamination der Glasoberfläche mit im Wasser gelösten Stoffen stattfindet. Besonders bevorzugt wird der Wasserdampf in Form von mit Wasser angereicherter Luft zugeführt.

Glasoberflächen weisen nach ihrer Herstellung und Lagerung unter normalen atmosphärischen Bedingungen insbesondere nach einiger Zeit an Luft eine Oberflächenenergie auf, die mit einem Kontaktwinkel von typischerweise 20° bis 60° einhergeht. Dieser Kontaktwinkel wird mit dem erfindungsgemäßen Verfahren deutlich erniedrigt und die Oberfläche damit hydrophilisiert.

Neben einem mit dem erfindungsgemäß erzielbaren niedrigen Kontaktwinkel des Glases mit Wasser ist auch eine homogene Ausbildung der Oberflächenenergie auf der Zylinder-Innenwandung von Vorteil. Ändert sich die Oberflächenenergie, so variiert auch der Reibwiderstand zum Reibpartner. Dies kann sogar so weit gehen, dass der Kolben bei einer gegebenen aufgewendeten Schubkraft blockiert. Selbst ein sich nur verändernder Widerstand beim Bewegen des Kolbens kann sehr nachteilig sein, etwa, wenn dies eine genaue Dosierung von Medikamenten mit einer Glasspritze erschwert. Gemäß der Erfindung ist daher vorgesehen, dass mit der Gasentladung, Plasma-, UV- oder Ozonbehandlung nicht nur der Kontaktwinkel mit Wasser herabgesetzt wird, sondern auch, dass der Kontaktwinkel des Glases mit Wasser entlang der axialen Richtung des Glaszylinders eine Variation aufweist, deren Größe durch die Gasentladung, Plasma-, UV- oder Ozonbehandlung herabgesetzt wird. Ein erfindungsgemäßer Glaszylinder zeichnet sich dabei im Allgemeinen dadurch aus, dass die Variation des Kontaktwinkels in Längsrichtung von Ende zu Ende des Glaszylinders weniger als 5° beträgt.

Mit der Erfindung ist dabei ein Glaszylinder für eine Kolben-Zylinder-Anordnung herstellbar, bei welchem die Oberfläche der Zylinder-Innenseite durch das Glas des Glaszylinders gebildet wird, und wobei der Kontaktwinkel dieser Oberfläche mit Wasser kleiner als 15° beträgt. Der Glaszylinder ist also, da die Oberfläche durch das Glas selbst gebildet wird, anders als bei bekannten Glasspritzen nicht beschichtet.

Grundsätzlich können alle möglichen Glasarten, wie Kalknatronglas, Alumosilikatglas, Borosilikatglas, Quarzglas, diverse Glaskeramiken und weitere verwendet werden. Besonders geeignet sind Borosilikatgläser.

Der Effekt der Hydrophilisierung, wie er durch die Erfindung mittels der Gasentladung hervorgerufen wird, wird durch die Beladung mit Wasser stabilisiert. Die Reaktion der Glasoberfläche mit Wassermolekülen im Anschluss an die Einwirkung der Gasentladung führt offensichtlich dazu, dass die reaktiven Zentren an der Oberfläche mit Wasser reagieren und dadurch abgesättigt werden. Dadurch kann eine Reaktion mit anderen Reaktionspartnern nicht oder allenfalls erschwert erfolgen. Vorzugsweise ist vorgesehen, die Beladung innerhalb von 60 Minuten nach Beendigung der Gasentladung zu beginnen. Diese Beladung kann insbesondere auch das Befüllen mit der Wirkstoff-Zubereitung umfassen.

Aus den Untersuchungen in J. Abenojar et al., International Journal of Adhesion & Adhesives 44 (2013) 1-8 ergibt sich, dass eine Plasmabehandlung einer Glasoberfläche mittels einer Plasmafackel eine Erhöhung von brückenbildendem Sauerstoff relativ zu nichtbrückenbildendem Sauerstoff mit sich bringt. Ein entsprechender Effekt ist auch bei der im Inneren des Glaszylinders hervorgerufenen Gasentladung, Plasma-, UV- oder Ozonbehandlung zu beobachten. Zusätzlich werden C-haltige Verbindungen, welche sich aus der Atmosphäre an die Glasoberfläche angelagert haben, aufgebrochen oder fragmentiert bzw. die Bindung zu den Silanolgruppen der Glasoberfläche aufgebrochen und anschließend via Luftströmung / Konvektion abtransportiert.

Bei der Oberflächenaktivierung des Glases mit UV-Licht oder Ozon erfolgt insbesondere Folgendes: Die UV-Photonen sind aufgrund ihrer hohen Energie in der Lage, chemische Bindungen in dem molekularen Netzwerk des Glases aufzubrechen. Die geöffneten Bindungsstellen sind bestrebt, schnellstmöglich wieder einen chemisch stabilen Zustand zu erreichen. Als Reaktionspartner dient hierzu beispielsweise der Sauerstoff aus der Atmosphäre oder Ozon, das insbesondere bei UV-Einstrahlung aus dem Umgebungssauerstoff gebildet werden kann. Die offenen Bindungen werden aus daraus gebildeten Atomen und Radikalen abgesättigt und es entstehen neue Verbindungen an der Glasoberfläche. Die Oberfläche erhält dadurch einen höheren polaren Charakter, was sich auch auf die Oberflächenenergie und damit den Kontaktwinkel gegenüber Wasser auswirkt.

Gemäß einer Ausführungsform der Erfindung ist daher vorgesehen, dass an der Oberfläche der Zylinder-Innenseite das Verhältnis von brückenbildendem Sauerstoff zu nicht brückenbildendem Sauerstoff gegenüber dem Wert dieses Verhältnisses im Inneren des Glases erhöht ist. Gemäß noch einer Ausführungsform der Erfindung ist an der Oberfläche der Zylinder-Innenseite das Verhältnis von Sauerstoff zu Silizium gegenüber dem Wert dieses Verhältnisses im Inneren des Glases erhöht. Auch dieser Effekt, welcher eine Steigerung des polaren Anteils der Oberflächenenergie bewirkt, wird im oben genannten Artikel beschrieben.

Aus T. Ishibe et al., "Absorption of nitrogen dioxide and nitric oxide by soda lime", British Journal of Anaesthesia 1995; 75:330-333 ist es weiterhin bekannt, dass Stickstoffmonoxid, NO und Stickstoffdioxid, NO₂, durch Glas absorbiert werden, wobei die Absorption von NO nur bei gleichzeitiger Präsenz von NO₂ stattfindet. Beide Substanzen werden bei einer Gasentladung in einem Stickstoff und Sauerstoff enthaltenden Gas, also insbesondere auch bei einer Gasentladung in Anwesenheit von Luft erzeugt. Daher ist gemäß noch einer Ausführungsform der Erfindung vorgesehen, dass das Glas an der Zylinder-Innenseite an der Oberfläche oder oberflächennah Stickoxide oder noch allgemeiner Stickstoffverbindungen enthält.

Unter Verwendung eines Glaszylinders wie er vorstehend charakterisiert wurde und wie er erfindungsgemäß herstellbar ist, kann nun eine Kolben-Zylinder-Anordnung mit dem Glaszylinder, sowie einem in den Glaszylinder eingesetzten Kolben, der auch als Stopfen bezeichnet wird, hergestellt werden, wobei der Kolben direkt auf dem Glas des Glaszylinders läuft und wobei zumindest die Lauffläche des Kolbens durch einen Kunststoff gebildet wird, wobei der Kunststoff der Lauffläche einen größeren Kontaktwinkel mit Wasser aufweist, als die mit dem Glas des Glaszylinders gebildete Oberfläche der Zylinder-Innenwandung. Typischerweise ist der Unterschied so groß, dass die Lauffläche des Kolbens als hydrophob und das Glas als Reibpartner als hydrophil charakterisiert werden kann.

Insbesondere können die Kontaktwinkel von Kunststoff der Lauffläche und Zylinder-Innenwandung mit Wasser eine Differenz von mindestens 60°, vorzugsweise mindestens 70° aufweisen. Durch die hohe Differenz lassen sich gute Gleiteigenschaften erreichen, ohne dass es eines Schmiermittels, wie etwa des sonst verwendeten Silikonöls, bedarf. Die Erfindung kann nun gemäß einer Weiterbildung auch dazu genutzt werden, eine bestimmte, vorgegebene Differenz der Kontaktwinkel von Lauffläche des Kolbens und Zylinder-Innenseite gezielt einzustellen, indem die Gasentladung, Plasma oder Ozonbehandlung so lange durchgeführt wird, bis der Kontaktwinkel der Glasoberfläche so weit abgesunken ist, dass die vorgegebene Differenz erreicht wird. Zudem wird dadurch auch ein über die Längsseite des Zylinders hinweg homogener Kontaktwinkel erreicht.

Generell ist das beschriebene Verfahren immer dann einsetzbar, wenn hydrophile Glasoberflächen herzustellen sind. Dies beschränkt sich nicht nur auf zylinderförmige Formkörper, sondern können in besonderen Ausführungsformen auch flache Formen sein, welche in besonderen Ausführungsformen anschließend auch geformt werden, beispielsweise durch Aufrollen eines Dünnglases.

In die vorzugsweise in Form einer Glasspritze ausgebildete Kolben-Zylinder-Anordnung kann dann bei zurückgezogenem Kolben eine flüssige Wirkstoff-Zubereitung aufgenommen und aufbewahrt werden. Auf diese Weise wird dementsprechend eine vorbefüllte Spritze bereitgestellt.

Besonders bei komplexen Wirkstoffen mit hohem Molekulargewicht, wie Insulin, Impfstoffen, Antikörpern, Blutprodukten, Hormonen, Zytokinen, sowie allgemein proteinbasierten Wirkstoffen besteht bei der Verwendung von Silikon zur Senkung der Reibung zwischen Kolben und Zylinder die Gefahr einer Agglomeration des jeweiligen Wirkstoffs an der silikonisierten Oberfläche oder die Bildung von gelösten Silikonpartikeln im Pharmazeutika. Gleiches gilt auch im Falle anderer organischer Schmiermittel. Mit der erfindungsgemäßen, insbesondere silikon- oder schmiermittelfreien Glasspritze wird dieses Problem beseitigt oder zumindest die Gefahr einer Agglomeration deutlich reduziert. Gemäß einer vorteilhaften Ausgestaltung der Erfindung ist daher eine Kolben-Zylinder-Anordnung in Form einer Glasspritze vorgesehen, in der eine flüssige Wirkstoff-Zubereitung aufgenommen ist, die einen Wirkstoff mit einem Molekulargewicht von zumindest 1000 Gramm/Mol enthält.

Die Erfindung wird nachfolgend anhand von Ausführungsbeispielen und unter Bezugnahme auf die beigeschlossenen Figuren näher erläutert.

### Kurzbeschreibung der Figuren

- Fig. 1: zeigt eine Vorrichtung zur Behandlung eines Glaszylinders mittels Korona- Entladung.
- Fig. 2: zeigt eine weitere Vorrichtung zur Behandlung eines Glaszylinders mittels einer Korona-Entladung oder einer anderen Form eines Plasmas.
- Fig. 3: ist ein Diagramm mit Messwerten des Kontaktwinkels an verschiedenen Längspositionen einer unbehandelten Glasspritze und einer mittels einer Gasentladung behandelten Spritze.
- Fig. 4: zeigt eine gefüllte Glasspritze.
- Fig. 5: zeigt eine Vorrichtung zur Behandlung eines Glaszylinders mittels einer Strahlungsquelle für ionisierende Strahlung.
- Fig. 6: ist ein Diagramm mit Messwerten des Kontaktwinkels an verschiedenen Längspositionen einer unbehandelten Glasspritze (Referenz) und von erfindungsgemäß behandelten Spritzen.
- Fig. 7: ist ein Diagramm mit Messwerten der Haft-Gleit-Reibungs-Kräfte für eine unbehandelte Glasspritze (Referenz) und für erfindungsgemäß behandelte Spritzen.
- Fig. 8: ist ein Balkendiagramm mit Messwerten des Kontaktwinkels für mittels Korona- Entladung behandelte Glasspritzen über verschiedene Lagerungszeiten.

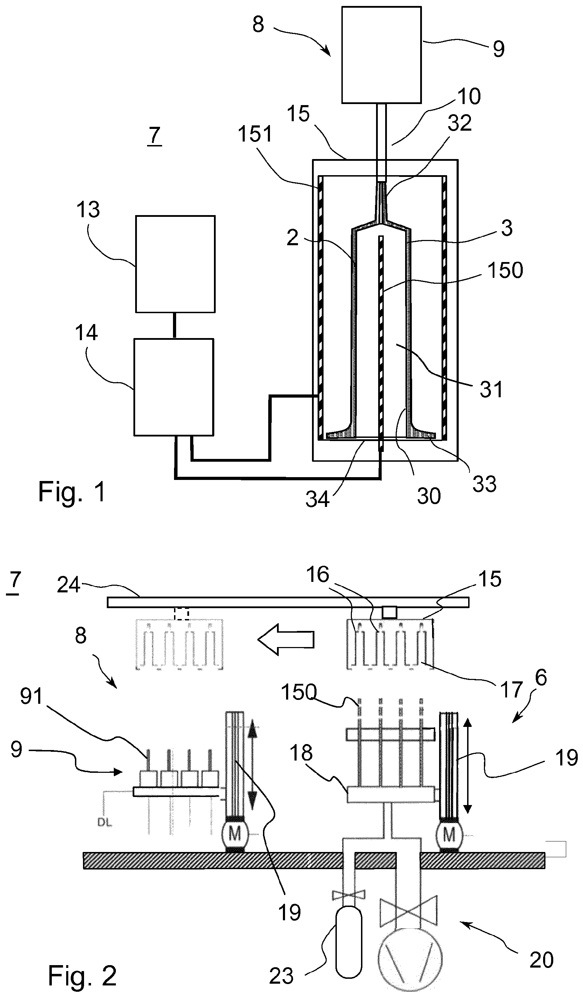

Allgemein, ohne Beschränkung auf das in Fig. 1 dargestellte Beispiel ist gemäß einem Aspekt der Erfindung eine Vorrichtung 7 zur Behandlung eines Glaszylinders 3 für eine Kolben-Zylinder-Anordnung zur Verminderung der Reibung des Kolbens an der Zylinder-Innenwandung vorgesehen, wobei die Vorrichtung 7 ausgebildet ist, im Glaszylinder 3 eine Korona-Entladung zu erzeugen. Die Vorrichtung 7 umfasst einen Hochfrequenzgenerator 13, einen Hochspannungstransformator 14 und eine Elektrodenstation 15.

Der Hochfrequenzgenerator 13 erzeugt ein Ausgangssignal, dessen Frequenz sich vorzugsweise je nach Widerstand automatisch im Bereich von 15-25 kHz anpasst und damit die Behandlungsleistung optimiert. Die Elektrodenstation 15 der Vorrichtung 7 umfasst mindestens eine Elektrode 150 und eine Gegenelektrode 151. Die Elektroden können für jede Applikation, beziehungsweise an verschiedene Formen von Glaszylindern 3 vorzugsweise speziell angepasst sein. Die funktionalisierende Korona-Entladung kann mit Luft unter Atmosphärendruck erzeugt werden. Die Elektrode 150 ist langgestreckt und in axialer Richtung des Glaszylinders 3 eingeführt. Die Gegenelektrode 150 ist röhrenförmig und umgibt den Glaszylinder 3. Dies sorgt für eine annähernd homogene Feldverteilung, so dass die aus Glas 2 bestehende Oberfläche der Innenseite 30 gleichmäßig der Korona-Entladung ausgesetzt wird. Um die Einwirkung der Korona-Entladung besonders gleichmäßig zu bekommen, kann während der Korona-Entladung auch eine Relativbewegung von Glaszylinder 3 und der Anordnung der Elektroden 150, 151 durchgeführt werden.

Bei dem gezeigten Beispiel ist der Glaszylinder 3 der Zylinder für eine Glasspritze 1. Dazu umfasst der Glaszylinder 3 eine Zylinder-Innenwandung 30 als Gleitfläche für einen Kolben 5, welche einen Zylinder-Innenraum 31 begrenzt, sowie ein Halteelement für eine Spritzennadel, typischerweise ausgebildet als Luer-Konus 32. Typischerweise weisen solche Glaszylinder 3 auch noch einen Flansch 33 an der Kolbeneinführöffnung 34 auf. Sofern die elektrische Entladung nicht an Luft und/oder nicht unter Atmosphärendruck vorgenommen wird, kann der Glaszylinder 3 auch an diesem Flansch 33 abgedichtet werden, um den Innenraum 31 zumindest teilweise zu evakuieren.

Die Korona-Entladung ist eine bevorzugte Ausführungsform der Erfindung. Außer Gasentladungen, wie Plasmabehandlung und speziell Korona-Entladungen gibt es auch andere Verfahren, welche das Glas 2 erfindungsgemäß an dessen Oberfläche verändern. Insbesondere können dies generell Verfahren sein, die Radikale erzeugen, welche dann mit dem Glas 2 reagieren. So kann Ozon durch eine Gasentladung in einem Sauerstoff enthaltenden Gas oder auch durch eine Bestrahlung mit ionisierenden Strahlen, wie insbesondere durch eine UV-Strahlung, beispielsweise aus dem Umgebungssauerstoff, erzeugt werden. Die Vorrichtung 7 umfasst gemäß einer Ausführungsform eine Beladungseinrichtung 8, um die zuvor mit der Gasentladung behandelte Innenseite 30 des Glaszylinders 3 mit Wasser zu beladen. Vorzugsweise ist eine Einrichtung 10 zur Anreicherung von Luft mit Wasser vorgesehen, um Luft mit einem hohen Feuchtigkeitsgrad bereitzustellen. Mittels einer Zuführeinrichtung 10 wird die Luft dann in den Innenraum 31 des Glaszylinders eingeführt. Anders als in Fig. 1 dargestellt kann die Beladungseinrichtung 8 auch eine separate Beladungsstation umfassen. Bei dieser Ausführungsform der Erfindung werden die Glaszylinder nacheinander erst einer Behandlungsstation zur Behandlung der Innenseite 30 mit der Gasentladung und anschließend einer Beladungsstation zur Beladung mit Wasser zugeführt werden.

Es hat sich aber auch gezeigt, dass die hydrophilen Eigenschaften der Glasoberfläche nach der erfindungsgemäßen Behandlung mit Gasentladung, wie etwa Plasma- oder Koronaentladung auch ohne vorübergehende Beladung stabil bleiben. Gemäß einer Ausführungsform kann daher die Beladungseinrichtung auch entfallen.

Gemäß einer weiteren, in Fig. 2 dargestellten Ausführungsform kann eine erfindungsgemäße Vorrichtung 7 verwendet werden, die eine Abwandlung der aus der WO 2012/097972 A1 bekannten Anordnung darstellt. Die Vorrichtung 7 umfasst zumindest eine Glaszylinder-Aufnahme 15 mit einer, vorzugsweise mehreren Aufnahme-Kammern 16, die wenigstens ein offenes Ende 17 aufweisen, durch welches ein zu behandelnder Glaszylinder 3 einführbar ist, so dass eine Öffnung des Glaszylinders 3 zum offenen Ende der Aufnahme-Kammer 16 weist. Ferner umfasst die Vorrichtung 7 ein verschiebbares Schließelement 18, welches mit der Glaszylinder-Aufnahme 15 über eine Hubvorrichtung 19 zusammenführbar ist, so dass beim Verschließen mittels der Hubvorrichtung 19 durch Bewegen des Schließelements 18 auf die Glaszylinder-Aufnahme 15 zu ein aufgenommener Glaszylinder 3 an einer Öffnung des Glaszylinders, vorzugsweise an dessen Kolbeneinführöffnung 34 gegenüber dem äußeren Atmosphärendruck abgedichtet wird. Zudem umfasst die Vorrichtung 7 eine Einrichtung 6 zum Erzeugen einer in dieser Ausführungsform gegenüber dem äußeren Atmosphärendruck abgeschlossenen Gasentladung, insbesondere einer Korona-Entladung oder einer anderen Form eines Plasmas mit zwei Elektroden, über die ein elektrisches oder elektromagnetisches Feld im Inneren eines aufgenommenen und abgedichteten Glaszylinders 3 erzeugbar ist. Ähnlich wie bei dem in Fig. 1 dargestellten Beispiel ist eine der Elektroden 150 dabei eine Innenelektrode, die durch die Kolbeneinführöffnung 34 axial in den Glaszylinder 3 eingeführt wird. Die Glaszylinder-Aufnahme 15 kann dann die Gegenelektrode bilden oder enthalten.

Die Einrichtung 6 zum Erzeugen einer Gasentladung, insbesondere einer Korona-Entladung oder eines Plasmas, beispielsweise in einer O₂, O₂/Ar oder O₂/N₂- Atmosphäre, umfasst eine Einrichtung 20 zum Evakuieren des Innenraums 31 über eine gasdichte Verbindung am Schließelement 18 sowie zumindest ein Behandlungswerkzeug, das für wenigstens einen Teilschritt der Oberflächen-Behandlung der Behälter mit dem offenen Ende 17 der Glaszylinder-Aufnahme 15 zusammenführbar ist. In einer besonderen Ausführungsform wird die Evakuierung nicht vollständig durchgeführt, oder es wird nach Evakuierung ein Prozessgas aus einem Prozessgas-Behälter 23 ein gewünschtes Prozessgas zugeführt, so dass der Gasdruck bei der Gasentladung mindestens 100 Millibar beträgt.

In einer weiteren bevorzugten Ausführungsform wird die Evakuierung bis in den Niederdruckbereich geführt, so dass der Gasdruck während der Oberflächenbehandlung höchstens 10 mbar, vorzugsweise aber mindestens 0,3 mbar beträgt.

Die Vorrichtung 7 weist weiterhin eine von der Einrichtung 6 zur Erzeugung der Gasentladung, insbesondere einer Korona-Entladung oder einer anderen Form eines Plasmas, räumlich getrennte Beladungseinrichtung 8 auf, mit welcher eine bestimmte Wassermenge auf der Glaszylinder-Innenwandung 30 aufbringbar ist. Mit einer Transporteinrichtung 24 wird die Glaszylinder-Aufnahme mit den darin aufgenommenen Glaszylindern mittels einer Transporteinrichtung 24 von der Einrichtung 6 zum Erzeugen einer Gasentladung, insbesondere einer Korona-Entladung oder einer anderen Form eines Plasmas, zu einer optionalen Beladungseinrichtung 8 transportiert. Auch für die Beladungseinrichtung 8 kann wie dargestellt eine Hubvorrichtung 19 vorgesehen sein, um das Beladungswerkzeug mit der Glaszylinder-Aufnahme 15 und den darin aufgenommenen Glaszylindern 3 zusammenzuführen. Die Beladungseinrichtung 8 umfasst wie bei der Ausführungsform der Fig. 1 eine Einrichtung 9 zur Anreicherung von Luft mit Wasser. Die angereicherte Luft wird über Gaslanzen 91 in die Glaszylinder eingeführt.

Die Einrichtung 6 kann sowohl zur Erzeugung einer Korona-Entladung, als auch einer anderen Form eines Plasmas, beispielsweise einer Glimmentladung, ausgebildet sein.

Der Verfahrensschritt der Behandlung mit der Gasentladung oder dem Plasma oder mit Ozon und der damit einhergehenden Aktivierung des Glases an der Innenseite 30 können kann dabei auch beliebig oft wiederholt werden, um gegebenenfalls eine Verbesserung des Ergebnisses zu erzielen. Gemäß der Erfindung wird eine Beladung mit Wasser durchgeführt, wobei das Aktivieren und anschließendes Beladen, vorzugsweise in Form von mit Wasser (WFI) angereicherter gereinigter Luft, mindestens zweimal hintereinander durchgeführt wird. Es ist ferner auch denkbar, die Verfahrensschritte in bestimmten Anwendungsfällen unabhängig voneinander als Einschritt-Prozess durchzuführen.

Die Gasentladung kann hinsichtlich der Leistung und Behandlungsdauer kontrolliert und gegebenenfalls nachgeregelt werden. Der Gleitkoeffizient auf der Oberfläche lässt sich über die Leistung der Quelle sowie die Verweilzeit in diesem Verfahrensschritt auf das jeweilige Behältnis, gezielt einstellen. Damit kann die Eigenschaft der Oberfläche auch an die Eigenschaften des später einzusetzenden Stopfens gezielt angepasst werden. Das Risiko einer Schichtablösung oder Partikelbildung ist bei der erfindungsgemäßen Behandlung nicht gegeben, wodurch eine Wechselwirkung mit dem Pharmazeutikum sowie das Kontaminationsrisiko für den Patienten auf ein Minimum reduziert ist.

Ein weiterer Vorteil der Erfindung ist die universelle Einsetzbarkeit des Verfahrens und der Vorrichtung für unterschiedlichste Verpackungsmaterialien. Das erfindungsgemäße Verfahren kann auch noch zusätzlich als kostengünstige und sichere Alternative an Stelle einer Standard- bzw. einer Einbrenn-Silikonisierung eingesetzt werden. Die Gleitfläche in Form einer Glasoberfläche wird in einem ersten Schritt mittels hochfrequenter Hochspannungsentladung gereinigt und aktiviert. In einem zweiten Prozessschritt wird die erste Gleitfläche mit Wasser, vorzugsweise WFI-Wasser in Form von Dampf in Kontakt gebracht bzw. beladen. Bei der Beladung mit angereicherter Luft ist die Wassermenge dabei so gering, dass ein zusätzlicher Trocknungsschritt entfallen kann. Die Beaufschlagung mit Wasser kann aber auch durch das Befüllen mit einer Wirkstoff-Zubereitung erfolgen.

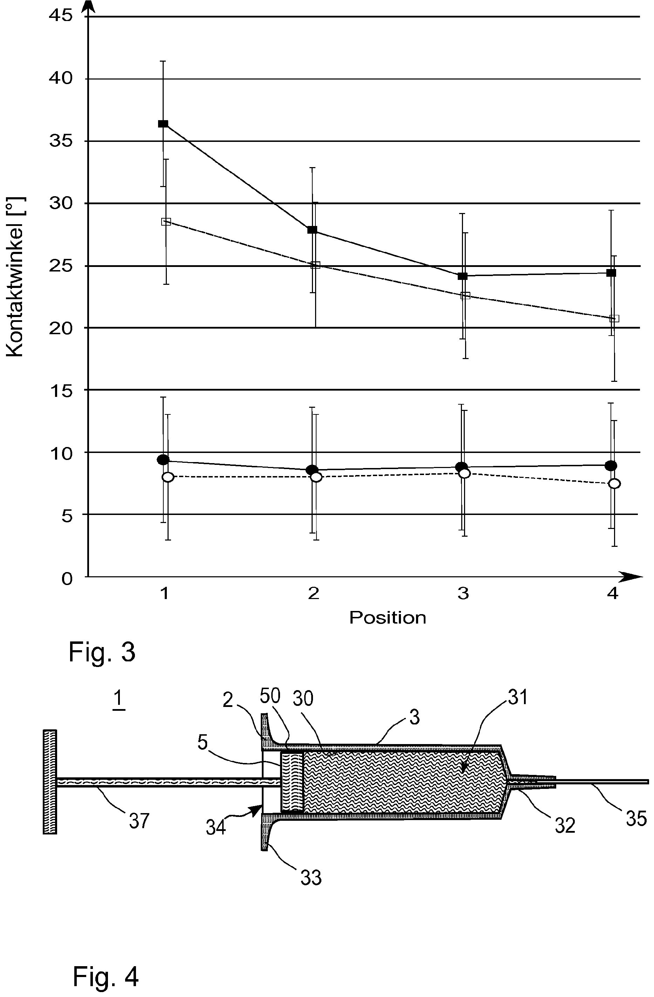

Neben einem mit dem erfindungsgemäß erzielbaren niedrigen Kontaktwinkel des Glases 2 mit Wasser ist auch eine homogene Ausbildung der Oberflächenenergie auf der Zylinder-Innenwandung 30 von Vorteil. Ändert sich die Oberflächenenergie, so variiert auch der Reibwiderstand zum Reibpartner. Dies kann sogar so weit gehen, dass der Kolben bei einer gegebenen aufgewendeten Schubkraft blockiert. Selbst ein sich nur verändernder Widerstand beim Bewegen des Kolbens 5 kann sehr nachteilig sein, etwa, wenn dies eine genaue Dosierung von Medikamenten mit einer Glasspritze 1, wie beispielsweise in Fig. 4 dargestellt, erschwert. Gemäß der Erfindung ist daher vorgesehen, dass mit der Gasentladung, Plasma-, UV- oder Ozonbehandlung nicht nur der Kontaktwinkel mit Wasser herabgesetzt wird, sondern auch, dass der Kontaktwinkel des Glases 2 mit Wasser entlang der axialen Richtung des Glaszylinders eine Variation aufweist, deren Größe durch die Gasentladung, Plasma- , UV- oder Ozonbehandlung herabgesetzt wird. Ein erfindungsgemäßer Glaszylinder 3 zeichnet sich dabei im Allgemeinen dadurch aus, dass die Variation des Kontaktwinkels in Längsrichtung von Ende zu Ende des Glaszylinders 3 weniger als 5° beträgt.

Ein Beispiel hierzu zeigt das Diagramm der Fig. 3. In diesem Diagramm sind die Messwerte des Kontaktwinkels des Glases 2 mit Wasser an vier Längspositionen einer unbehandelten Glasspritze 1 und einer mittels einer Gasentladung behandelten Glasspritze 1 eingetragen. Die Position 1 befindet sich dabei an der Kolbeneinführöffnung 34 der Glasspritze 1, wie zum Beispiel in Fig. 4 gezeigt. Die weiteren Positionen 2, 3 und 4 sind von Position 1 um 1,3 cm, 2,3 cm, 3,3 cm, beziehungsweise 4,3 cm beabstandet.

Die als Quadrate dargestellten Messwerte wurden an einer unbehandelten Glasspritze 1 gemessen. Als Kreise sind die Messwerte dargestellt, die an einer erfindungsgemäß innenseitig mittels einer Gasentladung behandelt wurden. Ausgefüllte Symbole stellen die Messwerte zu Beginn des Versuchs dar. Nach dieser ersten Messung wurden die Glasspritzen 1 für vier Tage in einer Schutzgasatmosphäre gelagert, danach wurde eine weitere Messung durchgeführt. Diese Messwerte an der unbehandelten und der erfindungsgemäßen Spritze 1 sind jeweils mit offenen Symbolen, also offenen Quadraten (unbehandelte Spritze) und offenen Kreisen (behandelte Spritze) dargestellt.

Die Messungen der Kontaktwinkel wurden mit dem Verfahren des ruhenden Tropfens ("sessile drop method") gemäß DIN 55660 mit folgenden geringfügigen Abweichungen von der Norm vorgenommen: Die Messungen fanden bei Raumtemperatur 22°C und 37% relativer Luftfeuchtigkeit (normgemäß sind 23°C, 50% rF). Wegen der Krümmung der Oberfläche wurden Tropfen mit einem Volumen von 1,5 Mikrolitern anstelle der vorgesehenen 2 Mikroliter verwendet.

Wie anhand der Messwerte augenfällig ist, führt die erfindungsgemäße Behandlung mit einer Gasentladung zu einer Absenkung des Kontaktwinkels mit Wasser auf unter 10°. Besonders vorteilhaft ist auch, dass der Effekt über den Lagerungszeitraum von 4 Tagen erhalten bleibt. Anders als bei der unbehandelten Spritze ist die Schwankung des Kontaktwinkels entlang der Längsrichtung des Glaszylinders 3 sehr klein. Bei der unbehandelten Spritze ist der Kontaktwinkel an der Kolbeneinführöffnung 34 am größten und sinkt zum anderen Ende, also zum Luer-Konus hin ab. Der Unterschied sowohl bei der gelieferten, als auch bei der 4 Tage gelagerten Spritze beträgt etwa 15°. Damit ist bereits die Variation des Winkels größer als der Kontaktwinkel des erfindungsgemäß behandelten Glaszylinders 3. Diese Variation ist im dargestellten Beispiel kleiner als 5°. Auch eine Variation von kleiner als 3° ist im Allgemeinen möglich, wie auch das Beispiel zeigt.

Der niedrige Kontaktwinkel des erfindungsgemäßen Beispiels wurde bereits bei einer einmaligen Behandlung erzielt. Generell ist es gemäß einer anderen Ausführungsform der Erfindung auch möglich, dass die Schritte des Hervorrufens einer Gasentladung und der Beladung mit Wasser mindestens einmal wiederholt werden.

Fig. 4 zeigt eine bevorzugte Anwendung einer erfindungsgemäßen Kolben-Zylinder-Anordnung 4 in Form einer Glasspritze 1. Insbesondere kann die Glasspritze 1 als vorgefüllte Spritze bereitgestellt werden, wobei bei zurückgezogenem Kolben 5 im Innenraum 31 des Glaszylinders 3 eine flüssige Wirkstoff-Zubereitung 11 aufgenommen ist. Die Glasspritze 1 kann auch bereits mit einer am Luer-Konus befestigten Kanüle 35 ausgestattet sein. Beispielsweise kann die Kanüle 35 eingeklebt werden. Die Befestigung der Kanüle 35 kann beispielsweise auch mit einem Verfahren erfolgen, wie es die WO 2012/034648 A1 beschreibt. Dabei wird die Kanüle in den Luer-Konus eingeschmolzen, so dass auch hier auf Kunststoffe durch Vermeidung einer Verklebung entfallen. Die Offenbarung der genannten Schrift wird bezüglich des Verfahrens des strahlungsgestützen Aufheizens des Glases und des Einschmelzens der Kanüle vollumfänglich auch zum Gegenstand der vorliegenden Anmeldung gemacht.

Die Besonderheit der Glasspritze 1 gemäß der Erfindung liegt nun darin, dass die Lauffläche 50 des Kolbens 5 direkt auf dem Glas 2 des Glaszylinders 3 läuft, also keine Schmiermittelschicht, insbesondere keine solche mit Silikon vorhanden ist. Die flüssige Wirkstoff-Zubereitung bildet dabei aufgrund des niedrigen Kontaktwinkels des behandelten Glases 2 einen Flüssigkeitsfilm auf der Oberfläche, auf dem die Lauffläche 50 gleitet. Die Lauffläche 50 des Kolbens 5 ist vorzugsweise aus einem Kunststoff, insbesondere einem Kunststoff, der einen größeren Kontaktwinkel zu Wasser aufweist, als die erfindungsgemäß behandelte Glasoberfläche. Die Kontaktwinkel von Kunststoff der Lauffläche 50 und Zylinder-Innenwandung 30 mit Wasser weisen dabei eine Differenz von mindestens 60°, vorzugsweise mindestens 70° auf. Gemäß einer Ausführungsform der Erfindung ist der Kunststoff der Lauffläche so gewählt, dass dessen Kontaktwinkel mit Wasser mindestens 70°, vorzugsweise mindestens 80° beträgt.

Wie gesagt, kann bei einem gegebenen Material der Lauffläche 50 auch eine bestimmte Kontaktwinkeldifferenz gezielt eingestellt werden, indem durch Dauer und/oder Intensität der Gasentladung, Plasma-, UV- oder Ozonbehandlung der Kontaktwinkel des Glases 2 so weit abgesenkt wird, dass die gewünschte Differenz erreicht wird.

Die Lauffläche 50 kann einstückig mit dem übrigen Teil des Kolbens 5 und der Schubstange 33 sein. Gegebenenfalls kann für die Lauffläche auch ein anderer Kunststoff eingesetzt werden, beispielsweise indem ein Stopfen bzw. Kolben 5 eingesetzt wird, bei dem zumindest die Oberfläche der Dichtung ein halogeniertes Polymer, vorzugsweise ein Fluorpolymer aufweist. Derartige Stopfen, die beispielsweise für bestimmte zu verabreichende Wirkstoffe wünschenswert sind, erweisen sich in Verbindung mit der erfindungsgemäßen Spritze und in Bezug auf die Gleit- und Abdichteigenschaften als besonders vorteilhaft.

Da keine Gefahr besteht, dass es zu einer Koagulation komplexerer Wirkstoffe unter dem Einfluss von Silikon kommt, wie sie bei silikonhaltigen Gleitfilmen vorhanden ist, ist die Erfindung auch besonders für solche Wirkstoffe geeignet. Insbesondere ist dieser Vorteil bei Wirkstoffen mit Molekulargewichten von 1000 Gramm pro Mol gegeben.

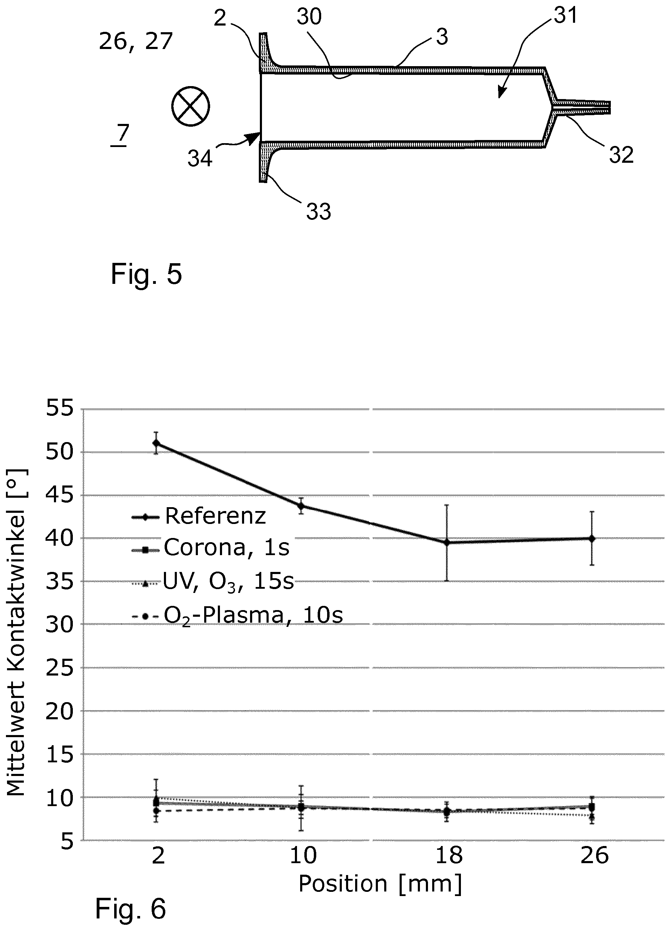

Es ist dem Fachmann ersichtlich, dass die Erfindung nicht auf die in den Figuren dargestellten speziellen Beispiele beschränkt ist, sondern vielmehr in vielfältiger Weise variiert werden kann. So wurde im Beispiel der Fig. 4 eine Spritze mit fest angebrachter Kanüle 35 gezeigt. Die Kanüle 35 kann aber auch erst bei Verwendung aufgesetzt werden. Auch werden Karpulen als Spritzen im Sinne der Erfindung erachtet. Fig. 5 zeigt Teile einer Vorrichtung 7 zur Behandlung eines Glaszylinders 3 mittels einer Strahlungsquelle 26 für ionisierende Strahlung, insbesondere einen UV-Strahler 27. Die Vorrichtung 7 umfasst im Ausführungsbeispiel einen UV-Strahler 27, beispielsweise in Form eines UV-Niederdruckstrahlers mit einer Röhre mit der Wellenlänge 185/254 Nanometer. Durch die UV-Bestrahlung des Glaszylinders 3, insbesondere der Glasspritze 1, wird Ozon im Innenraum des Glaszylinders erzeugt, welches dann auf die Innenwandung des Glaszylinders 3 für eine gewünschte Zeit ausgesetzt wird. Die Behandlung kann innerhalb oder außerhalb der Oberflächen des Glases 2 angeordnet sein. Das Verfahren der Aktivierung der Oberfläche initiiert über Ozon kann dabei auch beliebig wiederholt werden, um eine Verbesserung des Ergebnisses zu erhalten. Der Gleitkoeffizient auf der Oberfläche lässt sich über die Verweilzeit in diesem Verfahrensschritt auf dem jeweiligen Behältnis, dem Glas 2, dem Stopfen bzw. Kolben 5 und dem sonstigen Gegenstand gezielt einstellen. Das Risiko einer Schichtablösung oder Partikelbildung ist bei der erfindungsgemäßen Behandlung nicht gegeben, wodurch eine Wechselwirkung mit dem Pharmazeutikum sowie das Kontaminationsrisiko für den Patienten auf ein Minimum reduziert ist.

Ein weiterer Vorteil der Erfindung ist die universelle Einsetzbarkeit des Verfahrens und der Vorrichtung für unterschiedlichste Verpackungsmaterialien. Das erfindungsgemäße Verfahren kann auch noch zusätzlich als kostengünstige und sichere Alternative an Stelle einer Standard- bzw. einer Einbrenn-Silikonisierung eingesetzt werden.

Ozon kann auch durch Ionisierung von sauerstoffhaltigem Gas, beispielsweise durch Bestrahlung in einem vom Kolben 5 getrennten Reaktionsraum erzeugt und das ozonhaltige Gas aus dem Reaktionsraum dann in den Kolben 5 geleitet werden.

Wie bei der Ausführungsform mit der Behandlung durch eine Gasentladung wird die Hydrophilisierung durch die Einwirkung von Ozon durch eine Nachbeladung mit Wasser, etwa durch Wasserdampf stabilisiert.

Die erfindungsgemäßen Verfahren erlauben eine langzeitstabile Oberflächenbehandlung inklusive Transport und Lagerung bis zu einigen Jahren.

Nachfolgend sind drei erfindungsgemäße Behandlungen der Zylinder-Innenwandungen 30 von Glasspritzen 1 beschrieben.
Gemäß Ausführungsbeispiel 1 erfolgt eine Oberflächenmodifikation der Zylinder-Innenwandung 30 einer Glasspritze 1 mittels Korona-Entladung in der Luft. Dafür wird die Glasspritze 1 in einer Vorrichtung 7 gemäß Fig. 1 in die Gegenelektrode 151 aus Messing montiert und die Elektrode 150 aus Aluminium in den Glaszylinder 3 eingeführt. Die Korona-Entladung erfolgt bei einer effektiven Spannung von 3 kV und einer Frequenz von 15 kHz. Die Dauer kann variiert werden und beträgt in diesem Beispiel 1 Sekunde (getestet 0,1 bis 30 Sekunden).
Gemäß Ausführungsbeispiel 2
   erfolgt eine Oberflächenmodifikation der Zylinder-Innenwandung 30 einer Glasspritze 1 mittels UV/Ozon. Dafür wird die Glasspritze 1 in einer Vorrichtung 7 gemäß Fig. 5 der UV-Strahlung des UV-Strahlers 26 und Ozon für die Zeit zwischen 1 Sekunde und 10 Minuten ausgesetzt. Im konkreten Beispiel war die Behandlungsdauer 15 Sekunden.
Gemäß Ausführungsbeispiel 3
   erfolgt eine Oberflächenmodifikation der Zylinder-Innenwandung 30 einer Glasspritze 1 mittels Sauerstoff/Plasma. Dafür wird die Glasspritze 1 in einer Vorrichtung 7 gemäß Fig. 2 positioniert, wobei die Elektrode 150 im Hohlraum des Glaszylinders 3 angeordnet und die Glasspritze 1 abgedichtet wird. Das reaktive Gas wird durch die Elektrode 150 selbst eingeführt. Der Sauerstoff-Druck wird dabei auf 0,3 mbar eingestellt und das Plasma für 10 Sekunden gezündet bei einer Frequenz von 60 kHz und einer Stromstärke von 10 mA.

In Fig. 6 sind die Messwerte des Kontaktwinkels an vier verschiedenen Längspositionen einer unbehandelten Glasspritze (Referenz), einer 1 Sekunde mittels Korona-Entladung in der Luft behandelten Glasspritze 1 (gemäß Ausführungsbeispiel 1), einer 15 Sekunden mittels UV/Ozon behandelten Glasspritze 1 (gemäß Ausführungsbeispiel 2), und einer 10 Sekunden mittels Sauerstoff/Argon behandelten Glasspritze 1 (gemäß Ausführungsbeispiel 3), eingetragen. Dabei ist der Abstand vom Flansch (auch als Bund bezeichnet) an der Kolbeneinführöffnung 34 der jeweiligen Glasspritze 1 angegeben. Die erste Position befindet sich dabei 2 mm vom Flansch 33 beabstandet. Die weiteren Positionen sind bei einem Abstand von 10 mm, 18 mm und 26 mm vom Flansch 33 an der Kolbeneinführöffnung 34 angegeben. Die Messungen der Kontaktwinkel wurden mit dem oben zu Fig. 3 beschriebenen Verfahren vorgenommen.

Wie in Fig. 6 ersichtlich, beträgt der Kontaktwinkel der Zylinder-Innenwandung 30 der mittels Korona-Entladung behandelten Glasspritze 1 weniger als 10°.

Der Kontaktwinkel der Zylinder-Innenwandung 30 der mittels UV/Ozon behandelten Glasspritze 1 ist gemäß Fig. 6 ebenfalls auf einen Wert von weniger als 10° gefallen, homogen über die Spritzenlänge.

Wie in Fig. 6 gezeigt, ist auch der Kontaktwinkel der Zylinder-Innenwandung 30 der mittels Sauerstoff/Plasma behandelten Glasspritze 1 homogen über die Spritzenlänge kleiner als 10°.

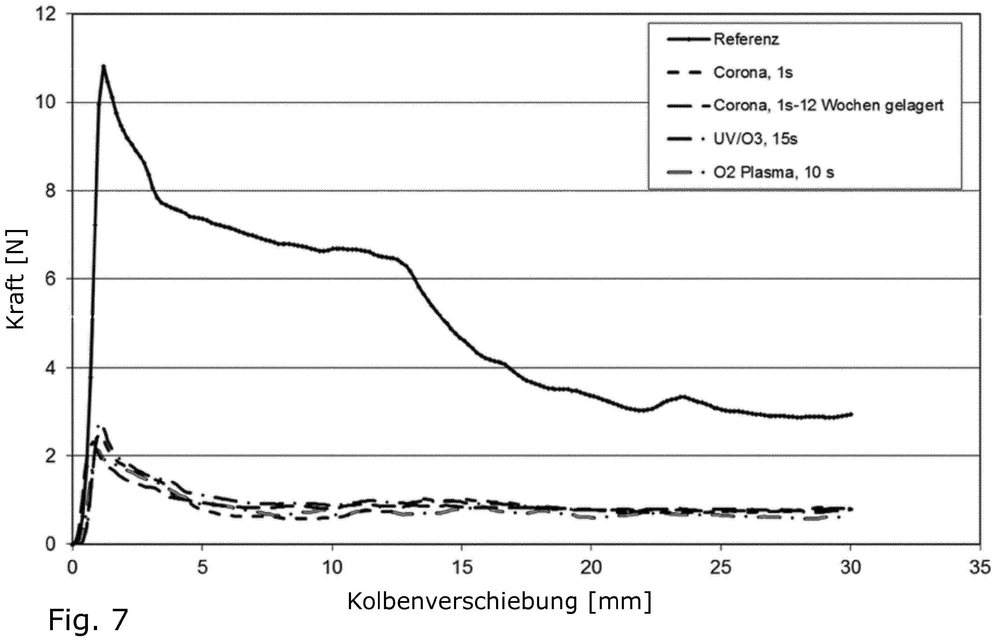

Fig. 7 ist ein Diagramm mit Messwerten der Haft-Gleit-Reibungs-Kräfte für eine unbehandelte Glasspritze (Referenz) und für erfindungsgemäß behandelte Glasspritzen 1. Wie in Fig. 7 ersichtlich, ist die Haft-Gleit-Reibungs-Kraft gemessen auf der 1 Sekunde mittels Korona-Entladung in der Luft behandelten Glasspritzen 1 (gemäß Ausführungsbeispiel 1) signifikant geringer als für die unbehandelte Referenz-Glasspritze, was auf eine homogene, superhydrophile Oberfläche zurückzuführen ist.

Nach der Korona-Entladung wurde die Glasspritzen 12 Wochen unter Raumtemperatur-Bedingungen eingelagert und die Haftgleitreibung erneut gemessen. Die Werte sind vergleichbar mit denen von frisch behandelten Spritzen, wie in Fig. 7 dargestellt.

Die Haft-Gleit-Reibungs-Kraft der 15 Sekunden mittels UV/Ozon behandelten Glasspritzen 1 (gemäß Ausführungsbeispiel 2), ist gemäß Fig. 7 ebenfalls signifikant niedriger als für die unbehandelte Referenz-Glasspritze.

Gleiches gilt für die 10 Sekunden mittels Sauerstoff/Argon behandelten Glasspritzen 1 (gemäß Ausführungsbeispiel 3), wie in Fig. 7 dargestellt.

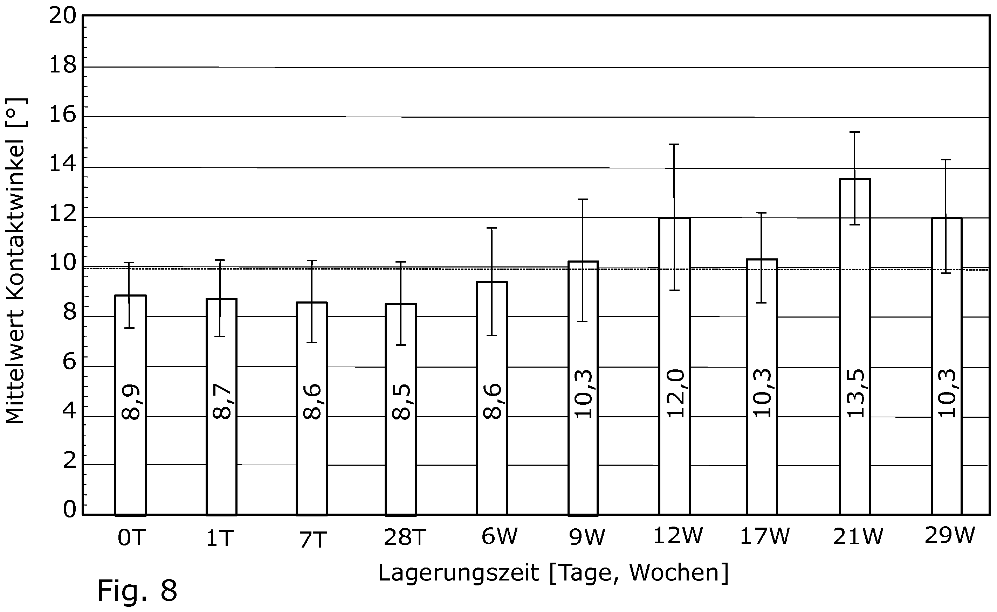

Um die Langzeitstabilität der Behandlung zu prüfen, wurden Glasspritzen 1 mittels Korana-Entladung (gemäß Ausführungsbeispiel 1) behandelt und anschließend unter Raumtemperatur eingelagert und der Kontaktwinkel nach Zeitabständen von 1 Tag, 7 Tagen, 28 Tagen, 6 Wochen, 9 Wochen, 12 Wochen, 17 Wochen, 21 Wochen und 29 Wochen Lagerzeit gemessen. Fig. 8 ist ein Balkendiagramm mit den Mittelwerten der Messungen des Kontaktwinkels für 10 mittels Korona-Entladung behandelte Glasspritzen pro Gruppe auf je 4 Positionen über die genannten Lagerungszeiten angegeben.

Wie aus Fig. 8 ersichtlich, bleibt der Kontaktwinkel über die bis zu 29 Wochen lange Lagerzeit stabil. Das ist signifikant länger als in der Literatur (bei S. Takeda, J. Non Cryst Solids, (249) 199, 41 - 46) beschrieben, in der nach ungefähr 8 Tagen der Kontaktwinkel von verschiedenen Glastypen wieder auf einen konstanten Wert angestiegen ist.

**Bezugszeichenliste**

| | |
|---|---|
| 1 | Glasspritze |
| 2 | Glas |
| 3 | Glaszylinder |
| 4 | Kolben-Zylinder-Anordnung |
| 5 | Kolben |
| 6 | Einrichtung zum Erzeugen einer Gasentladung |
| 7 | Vorrichtung zur Behandlung eines Glaszylinders 3 |
| 8 | Beladungseinrichtung |
| 9 | Einrichtung zur Anreicherung von Luft mit Wasser |
| 10 | Zuführeinrichtung |
| 11 | Wirkstoff-Zubereitung |
| 13 | Hochfrequenzgenerator |
| 14 | Hochspannungstransformator |
| 15 | Glaszylinder-Aufnahme |
| 16 | Aufnahme-Kammer |
| 17 | Offenes Ende von 16 |
| 18 | Schließelement |
| 19 | Hubvorrichtung |
| 20 | Einrichtung zum Evakuieren von 31 |
| 23 | Prozessgas-Behälter |
| 24 | Transporteinrichtung |
| 26 | Strahlungsquelle für ionisierende Strahlung |
| 27 | UV-Strahler |
| 30 | Zylinder-Innenwandung |
| 31 | Innenraum von 3 |
| 32 | Luer-Konus |
| 33 | Flansch |
| 34 | Kolbeneinführöffnung |
| 35 | Kanüle |
| 37 | Schubstange |
| 50 | Lauffläche von 5 |
| 91 | Gaslanzen |
| 150 | Elektrode |
| 151 | Gegenelektrode |

## Patentansprüche

1. Verfahren zur Behandlung eines Glaszylinders (3) für eine Kolben-Zylinder-Anordnung (4) zur Verminderung der Reibung des Kolbens (5) an der Zylinder-Innenwandung (30), bei welchem im von der Zylinder-Innenwandung (30) begrenzten Innenraum (31) die Oberflächenenergie des Glases (2) erhöht und dadurch der Kontaktwinkel des Glases (2) mit Wasser abgesenkt wird durch
- eine Gasentladung, welche auf das Glas (2) an der Zylinder-Innenwandung (30) einwirkt und durch ein elektrisches oder elektromagnetisches Feld erzeugt wird, oder
- durch Einwirkung von Ozon auf die Glasoberfläche (2), wobei nach der Einwirkung der Gasentladung, des Plasmas oder des Ozons das Glas (2) an der Zylinder-Innenseite (30) mit Wasser beaufschlagt wird,
wobei die Oberfläche der Zylinder-Innenseite (30) durch das Glas (2) des Glaszylinders (3) gebildet wird, und wobei der Kontaktwinkel dieser Oberfläche mit Wasser kleiner als 15° beträgt und die Variation des Kontaktwinkels des Glases mit Wasser in Längsrichtung von Ende zu Ende des Glaszylinders weniger als 5° beträgt.

2. Verfahren gemäß dem vorstehenden Anspruch, **dadurch gekennzeichnet, dass** das Beaufschlagen mit Wasser durch Befüllen mit einer wasserhaltigen Wirkstoff-Zubereitung erfolgt.

3. Verfahren gemäß einem der vorstehenden Ansprüche, **dadurch gekennzeichnet, dass** die Gasentladung in einem Sauerstoff enthaltenden Gas, insbesondere reinem Sauerstoff, einem Sauerstoff-Stickstoff-Gemisch, einem Sauerstoff-Edelgas-Gemisch oder Luft erfolgt.

4. Verfahren gemäß einem der vorstehenden Ansprüche, **dadurch gekennzeichnet, dass** das Ozon durch zumindest einen der Prozesse:
- eine Gasentladung in einem Sauerstoff enthaltenden Gas,
- eine Bestrahlung mit ionisierenden Strahlen,
- eine UV-Bestrahlung
erzeugt wird.

5. Verfahren gemäß einem der vorstehenden Ansprüche, **gekennzeichnet durch** zumindest eines der Merkmale:
- die Gasentladung erfolgt bei einem Gasdruck von mindestens 100 Millibar oder bei einem Gasdruck von höchstens 10 Millibar,
- die Gasentladung umfasst eine Korona-Entladung oder eine Plasmabehandlung.

6. Verfahren gemäß einem der vorstehenden Ansprüche, **gekennzeichnet durch** zumindest eines der folgenden Merkmale:
- der Kontaktwinkel des Glases mit Wasser entlang der axialen Richtung des Glaszylinders weist eine Variation auf, deren Größe durch die Gasentladung oder durch die Einwirkung von Ozon herabgesetzt wird,
- es wird eine Differenz der Kontaktwinkel von Lauffläche (50) des Kolbens (5) und der Zylinder-Innenseite (30) vorgegeben und die Gasentladung, oder die Einwirkung von Ozon wird so lange durchgeführt, bis der Kontaktwinkel des Glases so weit abgesunken ist, dass die vorgegebene Differenz erreicht wird.

7. Verfahren gemäß einem der vorstehenden Ansprüche, **dadurch gekennzeichnet, dass** die Schritte des Hervorrufens einer Gasentladung, oder der Einwirkung von Ozon und der Beladung mit Wasser mindestens einmal wiederholt werden.

8. Glaszylinder (3) für eine Kolben-Zylinder-Anordnung (4), herstellbar mit einem Verfahren gemäß einem der vorstehenden Ansprüche, wobei die Oberfläche der Zylinder-Innenseite (30) durch das Glas (2) des Glaszylinders (3) gebildet wird, und wobei der Kontaktwinkel dieser Oberfläche mit Wasser kleiner als 15° beträgt und die Variation des Kontaktwinkels des Glases mit Wasser in Längsrichtung von Ende zu Ende des Glaszylinders weniger als 5° beträgt, **gekennzeichnet durch** zumindest eines der folgenden Merkmale:
- an der Oberfläche der Zylinder-Innenseite (30) ist das Verhältnis von brückenbildendem Sauerstoff zu nicht brückenbildendem Sauerstoff gegenüber dem Wert dieses Verhältnisses im Inneren des Glases (2) erhöht,
- an der Oberfläche der Zylinder-Innenseite (30) ist das Verhältnis von Sauerstoff zu Silizium gegenüber dem Wert dieses Verhältnisses im Inneren des Glases (2) erhöht,
- das Glas (23) des Glaszylinders (3) an der Zylinder-Innenseite enthält zumindest eine Stickstoff-Verbindung.

9. Kolben-Zylinder-Anordnung (4) mit einem Glaszylinder (3) gemäß dem vorstehenden Anspruch, sowie einem in den Glaszylinder (3) eingesetzten Kolben (5), wobei der Kolben (5) direkt auf dem Glas (2) des Glaszylinders (3) läuft, wobei zumindest die Lauffläche (50) des Kolbens (5) durch einen Kunststoff gebildet wird, wobei der Kunststoff der Lauffläche (50) einen größeren Kontaktwinkel mit Wasser aufweist, als die mit dem Glas (2) des Glaszylinders (3) gebildete Oberfläche der Zylinder-Innenwandung (30).

10. Kolben-Zylinder-Anordnung (4) gemäß dem vorstehenden Anspruch, **gekennzeichnet durch** zumindest eines der folgenden Merkmale:
- die Kontaktwinkel von Kunststoff der Lauffläche (50) und Zylinder-Innenwandung (30) mit Wasser weisen eine Differenz von mindestens 60°, vorzugsweise mindestens 70° auf
- zumindest die Oberfläche der Dichtung weist ein halogeniertes Polymer, vorzugsweise ein Fluorpolymer auf.

11. Kolben-Zylinder-Anordnung (4) gemäß einem der zwei vorstehenden Ansprüche, in Form einer Glasspritze (1).

12. Kolben-Zylinder-Anordnung (4) gemäß dem vorstehenden Anspruch, **gekennzeichnet durch** einen in der Glasspritze (1) bei zurückgezogenem Kolben (5) aufgenommene flüssige Wirkstoff-Zubereitung (11), bevorzugt mit einem Wirkstoff mit einem Molekulargewicht von zumindest 1000 Gramm/Mol.

## Claims

1. A method for treating a glass cylinder (3) for a plunger-cylinder assembly (4) for reducing friction of the plunger (5) on the cylinder inner wall (30), wherein in the interior space (31) bounded by the cylinder inner wall (30) the surface energy of the glass (2) is increased and thereby the contact angle of the glass (2) with water is lowered by
- subjecting the glass (2) at the cylinder inner wall (30) to a gas discharge generated by an electrical or electromagnetic field; or
- subjecting the glass surface (2) to ozone, wherein after the subjecting to the gas discharge, the plasma or the ozone, the glass (2) on the inner surface (30) of the cylinder is exposed to water;
wherein the surface of the cylinder inner side (30) is defined by the glass (2) of the glass cylinder (3), and wherein the contact angle of this surface with water is less than 15°, and wherein the variation of the contact angle of the glass with water in the longitudinal direction from end to end of the glass cylinder is less than 5°.

2. The method according to the preceding claim, **characterised in that** the exposure to water is achieved by filling with a water-containing active-substance preparation.

3. The method according to any one of the preceding claims, **characterised in that** the gas discharge is performed in an oxygen-containing gas, in particular in pure oxygen or in an oxygen-nitrogen mixture or in a mixture of oxygen and noble gas or in air.

4. The method according to any one of the preceding claims, **characterised in that** the ozone is generated by at least one of the following processes:
- a gas discharge in an oxygen-containing gas;
- an irradiation with ionizing radiation;
- a UV irradiation.

5. The method according to any one of the preceding claims, **characterised by** at least one of the following features:
- the gas discharge is performed at a gas pressure of at least 100 millibar or at a gas pressure of at most 10 millibar;
- the gas discharge comprises a corona discharge or a plasma treatment.

6. The method according to any one of the preceding claims, **characterised by** at least one of the following features:
- the contact angle of the glass with water along the axial direction of the glass cylinder exhibits a variation, the magnitude of which is reduced by the gas discharge or by the subjecting to ozone;
- a differential between the contact angles of a sliding surface (50) of the plunger (5) and of the cylinder inner surface (30) is predefined, and the gas discharge or the subjecting to ozone is continued until the contact angle of the glass is reduced such that the predefined differential is achieved.

7. The method according to any one of the preceding claims, **characterised in that** the steps of generating a gas discharge or of subjecting to ozone and of exposing to water are repeated at least once.

8. A glass cylinder (3) for a plunger-cylinder assembly (4), producible by a method according to any one of the preceding claims, wherein the surface of the cylinder inner side (30) is defined by the glass (2) of the glass cylinder (3), and wherein the contact angle of said surface with water is less than 15°, and wherein the variation of the contact angle of the glass with water in the longitudinal direction from end to end of the glass cylinder is less than 5°,
**characterised by** at least one of the following features:
- at the surface of the cylinder inner side (30), the ratio of bridging oxygen to non-bridging oxygen is increased relative to the value of this ratio in the interior of the glass (2);
- at the surface of the cylinder inner side (30), the ratio of oxygen to silicon is increased relative to the value of this ratio in the interior of the glass (2);
- the glass (23) of the glass cylinder (3) at the cylinder inner surface contains at least one nitrogen compound.

9. A plunger-cylinder assembly (4) comprising a glass cylinder (3) according to the preceding claim, and a plunger (5) inserted into the glass cylinder (3), wherein the plunger (5) slides directly on the glass (2) of the glass cylinder (3); wherein at least a sliding surface (50) of the plunger (5) is made of a plastic material; wherein the plastic material of the sliding surface (50) exhibits a larger contact angle with water than the surface of the cylinder inner wall (30) defined by the glass (2) of the glass cylinder (3).

10. The plunger-cylinder assembly (4) according to the preceding claim, **characterised by** at least one of the following features:
- the contact angles with water of the plastic material of the sliding surface (50) and of the cylinder inner wall (30) exhibit a differential of at least 60°, preferably at least 70°;
- at least the surface of the seal comprises a halogenated polymer, preferably a fluoropolymer.

11. The plunger-cylinder assembly (4) according to any one of the two preceding claims, in the form of a glass syringe (1).

12. The plunger-cylinder assembly (4) according to the preceding claim, **characterised by** a liquid active-substance preparation (11) received in the glass syringe (1) when the plunger (5) is retracted, preferably including an active substance having a molecular weight of at least 1000 g/mol.

## Revendications

1. Procédé de traitement d'un cylindre de verre (3) destiné à un ensemble piston-cylindre (4) aux fins de réduire la friction du piston (5) sur la paroi interne de cylindre (30), selon lequel l'énergie superficielle du verre (2) est augmentée dans l'espace intérieur (31) délimité par la paroi interne de cylindre (30), et de ce fait l'angle de contact du verre (2) avec l'eau est réduit en raison
- d'une décharge gazeuse qui agit sur le verre (2) sur la paroi interne de cylindre (30) et est produite par un champ électrique ou électromagnétique, ou
- en raison de l'action d'ozone sur la surface de verre (2), sachant qu'après l'action de la décharge gazeuse, du plasma ou de l'ozone, le verre (2) est soumis à l'action de l'eau sur la face interne de cylindre (30),
la surface de la face interne de cylindre (30) étant formée par le verre (2) du cylindre de verre (3), et l'angle de contact de cette surface avec l'eau étant inférieur à 15°, et la variation de l'angle de contact du verre avec l'eau, dans la direction longitudinale de bout-en-bout du cylindre de verre (3), étant inférieure à 5°.

2. Procédé selon la revendication précédente, **caractérisé en ce que** la sollicitation avec de l'eau s'effectue par remplissage avec une préparation aqueuse d'une substance active.

3. Procédé selon une des revendications précédentes, **caractérisé en ce que** la décharge gazeuse a lieu dans un gaz contenant de l'oxygène, en particulier dans de l'oxygène pur, un mélange oxygèneazote, un mélange oxygène-gaz rare ou dans l'air.

4. Procédé selon une des revendications précédentes, **caractérisé en ce que** l'ozone est produit par au moins un des processus suivants :
- une décharge gazeuse dans un gaz contenant de l'oxygène,
- une irradiation avec des rayons ionisants,
- une irradiation UV.

5. Procédé selon une des revendications précédentes, **caractérisé en ce qu'**il présente au moins une des caractéristiques suivantes :
- la décharge gazeuse a lieu à une pression du gaz d'au moins 100 millibars ou à une pression du gaz d'au maximum 10 millibars,
- la décharge gazeuse comporte une décharge corona ou un traitement au plasma.

6. Procédé selon une des revendications précédentes, **caractérisé en ce qu'**il présente au moins une des caractéristiques suivantes :
- l'angle de contact du verre avec l'eau, le long de la direction axiale du cylindre de verre, présente une variation dont l'amplitude est réduite par la décharge gazeuse ou par l'action d'ozone,
- une différence entre les angles de contact de la surface de glissement (50) du piston (5) et de la face interne de cylindre (30) est prédéfinie, et la décharge gazeuse ou l'action de l'ozone est effectuée jusqu'à ce que l'angle de contact du verre ait diminué de manière à obtenir la différence prédéfinie.

7. Procédé selon une des revendications précédentes, **caractérisé en ce que** les étapes de déclenchement d'une décharge gazeuse ou de l'action d'ozone et de chargement en eau sont répétées au moins une fois.

8. Cylindre de verre (3) destiné à un ensemble piston-cylindre (4), pouvant être fabriqué avec un procédé selon une des revendications précédentes, dans lequel la surface de la face interne de cylindre (30) est formée par le verre (2) du cylindre de verre (3), et l' angle de contact de cette surface avec l'eau est inférieur à 15°, et la variation de l'angle de contact du verre avec l'eau, dans la direction longitudinale de bout-en-bout du cylindre de verre (3), est inférieure à 5°, **caractérisé en ce qu'**il présente au moins une des caractéristiques suivantes :
- à la surface de la face interne de cylindre (30), le rapport entre l'oxygène pontant et l'oxygène non pontant est augmenté en comparaison avec la valeur de ce rapport à l'intérieur du verre (2),
- à la surface de la face interne de cylindre (30), le rapport entre l'oxygène et le silicium est augmenté en comparaison avec la valeur de ce rapport à l'intérieur du verre (2),
- le verre (23) du cylindre de verre (3), sur la face interne de cylindre, contient au moins un composé azoté.

9. Ensemble piston-cylindre (4) comprenant un cylindre de verre (3) selon la revendication précédente, et comprenant un piston (5) inséré dans le cylindre de verre (3), le piston (5) se déplaçant directement sur le verre (2) du cylindre de verre (3), sachant qu'au moins la surface de glissement (50) du piston (5) est constituée d'une matière plastique, la matière plastique de la surface de glissement (50) présentant un angle de contact avec l'eau qui est plus grand que celui de la surface de la paroi interne de cylindre (30) formée avec le verre (2) du cylindre de verre (3).

10. Ensemble piston-cylindre (4) selon la revendication précédente, **caractérisé en ce qu'**il présente au moins une des caractéristiques suivantes :
- les angles de contact de la matière plastique de la surface de glissement (50) et de la paroi interne de cylindre (30) avec l'eau présentent une différence d'au moins 60°, de préférence d'au moins 70°,
- au moins la surface du joint d'étanchéité comporte un polymère halogéné, de préférence un fluoropolymère.

11. Ensemble piston-cylindre (4) selon une des deux revendications précédentes, se présentant sous la forme d'une seringue en verre (1).

12. Ensemble piston-cylindre (4) selon la revendication précédente, **caractérisé en ce qu'**il présente une préparation aqueuse de substance active (11), comportant de préférence une substance active ayant un poids moléculaire d'au moins 1000 grammes/mol, contenue dans la seringue en verre (1) lorsque le piston (5) est rétracté.
